# EUROPEAN PATENT SPECIFICATION

(11) **EP 3 706 686 B1**
(45) Date of publication and mention of the grant of the patent: **07.06.2023**
(21) Application number: 18807744.0
(22) Date of filing: 12.10.2018
(51) Int. Cl.: A61F 13/00, A61M 1/00, A61F 13/02

(54) **MULTI-MODULE DRESSING AND THERAPY METHODS**
MULTIMODULVERBAND UND THERAPIEVERFAHREN
PANSEMENT MULTI-MODULE ET PROCÉDÉS DE THÉRAPIE

(30) Priority: 09.11.2017 US 201762584045 P
(43) Date of publication of application: 16.09.2020
(73) Proprietor: KCI Licensing, Inc., San Antonio, TX 78265-9508 (US)
(72) Inventor: POOLE, Leanna, Rawtenstall BB4 8JA (GB); BOLTON, Rachel, Halifax, West Yorkshire HX3 6JQ (GB); HILL, Clinton, Colne, Lancashire BB8 9BZ (GB)
(74) Representative: Simmons & Simmons
(86) International application number: PCT/US2018/055687
(87) International publication number: WO 2019/094147

(56) References cited:
- WO-A1-2015/173547
- US-A1- 2014 188 058
- US-A1- 2014 350 494
- US-A1- 2015 119 833
- US-A1- 2017 079 846

## Description

### RELATED APPLICATIONS

The present invention claims the benefit, under 35 use § 119(e), of the filing of U.S. Provisional Patent Application serial number 62/584,045, entitled "Multi-Module Dressing and Therapy Methods," filed November 9, 2017.

### TECHNICAL FIELD

The invention set forth in the appended claims relates generally to tissue treatment systems and more particularly, but without limitation, to dressings for application to a tissue site, to systems including such dressings.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

US2014/350494 and US2015/119833 disclose negative-pressure therapy systems.

Systems for providing therapy to a tissue site, are set forth in the appended claims. Illustrative embodiments are also provided to enable a person skilled in the art to make and use the claimed subject matter.

A system comprises a first manifold module. The first manifold module comprises a first manifold layer and a first silicone adhesive layer. The first manifold module may further comprise a first perforated substrate and a first acrylic adhesive layer adhering the first manifold layer to a first surface of the first perforated substrate. The first silicone adhesive layer may be disposed on a second surface of the first perforated substrate. The system also comprises a second manifold module configured to be releasably secured to the first manifold module by the first silicone adhesive layer. The second manifold module comprises a second manifold layer and a second silicone adhesive layer. The second manifold module may further comprise a second perforated substrate and a second acrylic adhesive layer adhering the second manifold layer to a first surface of the second perforated substrate. The second silicone adhesive layer may be disposed on a second surface of the second perforated substrate. The first and second manifold modules each comprise a plurality of fluid pathways to distribute reduced pressure from a reduced-pressure source to a tissue site.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide negative-pressure therapy in accordance with this specification;
Figure 2 is a schematic diagram illustrating additional details that may be associated with an example manifold module used in some embodiments of the therapy system of Figure 1; and
Figure 3 is schematic diagram illustrating additional details that may be associated with an example tissue interface used in some embodiments of the therapy system of Figure 1.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

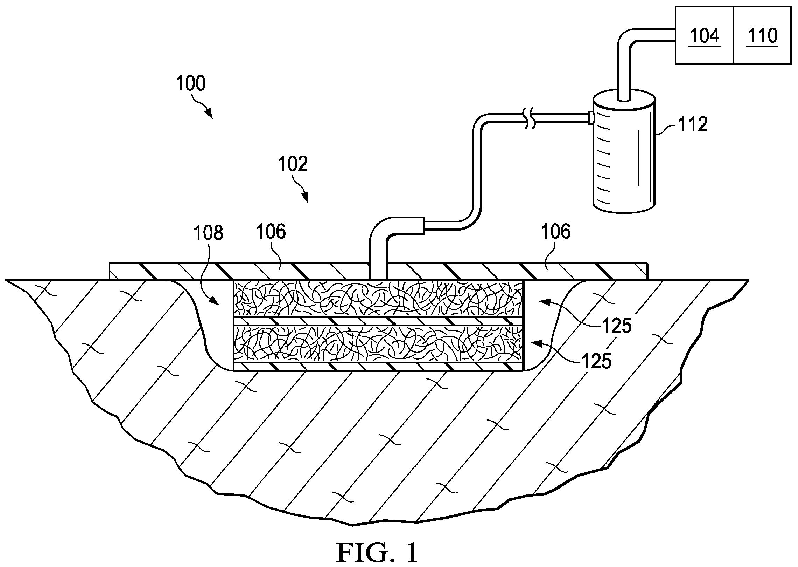

Figure 1 is a simplified functional block diagram of an example embodiment of a therapy system 100 that can provide negative-pressure therapy in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, and may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a tissue interface 108. A regulator or a controller, such as a controller 110, may also be coupled to the negative-pressure source 104.

In some embodiments, a dressing interface may facilitate coupling the negative-pressure source 104 to the dressing 102. For example, such a dressing interface may be the SENSAT.R.A.C.^{™} Dressing available from Acelity L.P. of San Antonio, Texas. The therapy system 100 may optionally include a fluid container, such as a container 112, coupled to the dressing 102 and to the negative-pressure source 104.

Additionally, the therapy system 100 may include sensors to measure operating parameters and provide feedback signals to the controller 110 indicative of the operating parameters. As illustrated in Figure 1, for example, the therapy system 100 may include a pressure sensor, an electric sensor, or both, coupled to the controller 110. The pressure sensor may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the negative-pressure source 104 may be directly coupled to the controller 110, and may be indirectly coupled to the dressing 102 through the container 112.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of a fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller 110 and other components into a therapy unit. A negative-pressure supply may also have one or more supply ports configured to facilitate coupling and de-coupling the negative-pressure supply to one or more distribution components.

The tissue interface 108 can be generally adapted to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

The tissue interface 108 comprise or consist of a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site. In some illustrative embodiments, the pathways of a manifold may be interconnected to improve distribution or collection of fluids across a tissue site.

The tissue interface 108 comprises two or more manifold modules 125. For example, in various embodiments, the tissue interface 108 may include two, three, four five, six, seven, eight, nine, ten, or more manifold modules 125, according to the needs of a prescribed therapy. The manifold modules 125 are releasably secured to each other to form the tissue interface 108. The term "releasably secured" in this context broadly refers to a configuration in which two or more manifold modules are physically engaged or coupled such that those two or more manifold modules may be physically disengaged, for example, uncoupled. In some embodiments, the two or more manifold modules 125 that have been disengaged may, after being disengaged, be physically re-engaged. In some embodiments according to the invention, two or more manifold modules 125 are releasably secured via adhesive.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least 300 g/m² per twenty-four hours in some embodiments. In some example embodiments, the cover 106 may be a polymer drape, such as a polyurethane film, that is permeable to water vapor but impermeable to liquid. The cover may have a thickness in the range of 25-50 microns. For permeable materials, the permeability generally may be low enough that a desired negative pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

A controller, such as the controller 110, may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. The controller 110 is also preferably configured to receive one or more input signals, such as a feedback signal, and programmed to modify one or more operating parameters based on the input signals.

Sensors, such as the pressure sensor or the electric sensor, are generally known in the art as any apparatus operable to detect or measure a physical phenomenon or property, and generally provide a signal indicative of the phenomenon or property that is detected or measured. For example, the pressure sensor and the electric sensor may be configured to measure one or more operating parameters of the therapy system 100. In some embodiments, the pressure sensor may be a transducer configured to measure pressure in a pneumatic pathway and convert the measurement to a signal indicative of the pressure measured. In some embodiments, for example, the pressure sensor may be a piezoresistive strain gauge. The electric sensor may optionally measure operating parameters of the negative-pressure source 104, such as the voltage or current, in some embodiments. Preferably, the signals from the pressure sensor and the electric sensor are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

### Manifold Modules-Manifold Layer

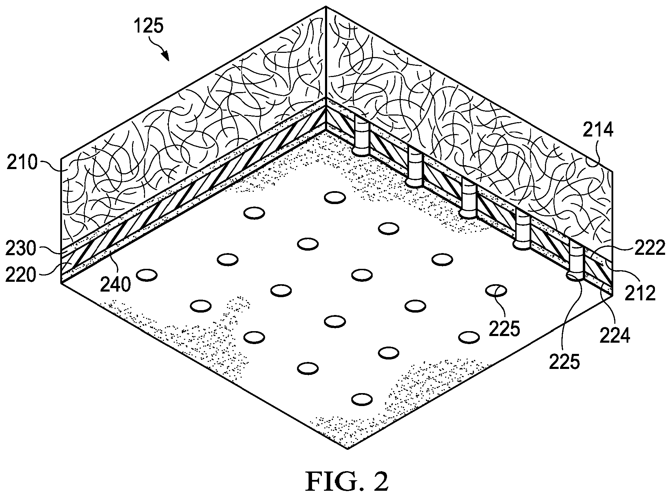

Figure 2 is a perspective view of an example of one of the manifold modules 125, illustrating additional details that may be associated with some embodiments. The manifold modules 125 each include a manifold layer 210. In various embodiments, the manifold layer 210 may take any suitable form, and may have a suitable size, shape, or thickness depending on a variety of factors, such as the type of treatment being implemented or the nature and size of a tissue site. For example, the size and shape of the manifold layer 210 may be adapted to the contours of deep and irregular shaped tissue sites and/or may be configured so as to be adaptable to a given shape or contour.

In some embodiments, the manifold layer 210 may comprise or consist of a manifold having two substantially planar surfaces and a depth or thickness orthogonal to the planar surfaces. For example, the manifold layer 210 may comprise a first surface 212 and a second surface 214. The first surface 212 and/or second surface 214 may have a surface area from about 1 cm² to about 400 cm², or from about 2 cm² to about 200 cm², or from about 4 cm² to about 100 cm². In various embodiments, the first surface 212 and/or the second surface 214 may have any suitable shape, examples of which include but are not limited to, triangles, squares, rectangles, ellipses, circles, ovals, and various polygons having four, five, six, seven, eight, or more sides. The shape and area of the first surface 212 and the second surface 214 may be customized to the location and type of tissue site onto which the tissue interface 108 is to be applied. In some embodiments, the manifold layer 210 may have a thickness from about 10 mm to about 500 mm, for example, from about 10 mm to about 100, or from about 100 mm to about 200 mm, or from about 200 mm to about 300 mm, or from about 300 mm to about 400 mm, or from about 400 mm to about 500 mm.

In some embodiments, the manifold layer 210 may comprise a porous foam material having interconnected cells or pores. For example, the manifold layer 210 may comprise cellular foam, for example, open-cell foam such as reticulated foam. Liquids, gels, and other foams may also include or be cured to include apertures and fluid pathways. In some embodiments, the manifold layer 210 may comprise projections that form interconnected fluid pathways. For example, the manifold layer 210 may be molded to provide surface projections that define interconnected fluid pathways.

In some embodiments, the foam may have an average pore size that varies according to needs of a prescribed therapy. For example, in some embodiments, the manifold layer 210 may be foam having pore sizes in a range of 400-600 microns. In some embodiments, the manifold layer 210 may have a tensile strength that also varies according to needs of a prescribed therapy.

In some embodiments, the foam material of the manifold layer 210 may be characterized with respect to density. In some embodiments, the manifold layer 210 may be characterized as a relatively dense material. For example, in various embodiments, the manifold layer 210 may have a density of from about 24 kg/m³ to about 125 kg/m³ or from about 24 kg/m³ to about 72 kg/m³.

In some embodiments, the manifold layer 210 may be hydrophobic. In some an embodiments, the hydrophobic characteristics may prevent the foam from directly absorbing fluid, such as wound exudate, but may allow the fluid to pass through a fluid pathway. For example, in some embodiments, the foam may be polyurethane foam, a silicone foam, a polyether block amide foam, such as PEBAX^{®}, an acrylic foam, a polyvinyl chloride (PVC) foam, a polyolefin foam, a polyester foam, a polyamide foam, a thermoplastic elastomer (TPE) foam such as a thermoplastic vulcanizate (TPV) foam, or another crosslinking elastomeric foam such as foams formed from styrene-butadiene rubber (SBR) and ethylene propylene diene monomer (EPDM) rubber. For example, the manifold layer 210 may comprise a hydrophobic, open-cell foam. In one non-limiting example, the manifold layer 210 may comprise an open-cell, reticulated polyurethane foam such as the foam employed in the V.A.C.^{®} GRANUFOAM^{™} Dressing or the foam employed in the V.A.C. VERAFLO^{™} Dressing, both available from available from Acelity L.P., Inc. of San Antonio, Texas.

In other embodiments, the manifold layer 210 may be hydrophilic. In some embodiments, the hydrophillic characteristics may be effective to wick fluid while also continuing to distribute negative pressure to the tissue site. In some embodiments, the wicking properties of the manifold layer 210 may draw fluid away from the tissue site by capillary flow or other wicking mechanisms. An example of a hydrophilic foam may include a polyvinyl alcohol or polyether, open-cell foam. Other foams that may exhibit hydrophilic characteristics include hydrophobic foams that have been treated or coated to provide hydrophilicity. For example, the manifold layer 210 may be a treated open-cell polyurethane foam. In one non-limiting example, the manifold layer 210 may comprise a polyvinyl alcohol, open-cell foam such as the foam employed in the V.A.C. WHITEFOAM^{™} Dressing available from Acelity L.P., Inc. of San Antonio, Texas.

### Non-Adherent Layer

In some embodiments, the manifold modules 125 may include a non-adherent layer 220. The term "non-adherent" is intended to refer broadly to a substrate that generally exhibits little tendency to adhere or stick to proximate tissue.

In various embodiments, the non-adherent layer 220 may comprise any suitable material and configuration. For example, in various embodiments, the non-adherent layer 220 may be a woven material or, alternatively, a nonwoven material such as a film. The non-adherent layer 220 may have a suitable thickness, for example, in the range of from about 10 µm to about 1000 µm. The non-adherent layer 220 may comprise a first surface 222 and a second surface 224.

In some embodiments, the non-adherent layer 220 may be formed from a generally flexible material. Non-limiting examples of such materials include thermoplastic materials, such as acrylics, acrylates (for example, ethyl methacrylate (EMA)), and thermoplastic elastomers such as nylon, styrene ethylene butene styrene (SEBS) and other block copolymers; polyether block polyamide (PEBAX); silicone elastomers; poly caprolactam; poly lactic acid; and polyolefins, such as polyethylene and polypropylene. In one non-limiting example, the non-adherent layer 220 may comprise a nylon film.

In some embodiments, at least a portion of the non-adherent layer 220 may be configured so as to be permeable to fluids. For example, in some embodiments, the non-adherent layer 220 may be characterized as porous, as perforated, or both. For example, the non-adherent layer 220 may comprise apertures 225 extending there-through so as to allow communication through the non-adherent layer 220. In such embodiments, the plurality of apertures 225 may have an average pore size in the range of from about 200 µm to about 3000 µm. In such embodiments, the plurality of apertures 225 may be present in the non-adherent layer 220 at a pore density in the range from about 2 pores/cm² to about 10 pores/cm².

In some embodiments, the non-adherent layer 220 may be generally configured to cover a surface of the manifold layer 210. For example, in the embodiment of Figure 2, the second surface 224 of the non-adherent layer 220 may cover or be applied to or engaged with the first surface 212 of the manifold layer 210.

### Manifold Modules- Adhesives

Two or or more of the manifold modules 125 include two or more adhesive layers. For example, the manifold modules 125 may include a first adhesive layer 230 and a second adhesive layer 240.

In some embodiments, not according to the invention the first adhesive layer 230 may couple the manifold layer 210 to the non-adherent layer 220. In some embodiments, not according to the invention the first adhesive layer 230 may be coupled directly to the manifold layer 210, the non-adherent layer 220, or both. In some embodiments, not according to the invention the first adhesive layer 230 may be coextensive with the manifold layer 210, the non-adherent layer 220, or both. In some embodiments, not according to the invention the first adhesive layer 230 may have a thickness from about 10 microns (µm) to about 1000 microns (µm). Additionally or alternatively, in some embodiments, not according to the invention the first adhesive layer 230 may have a coating weight from about 15 gsm to about 70 gsm.

In some embodiments not according to the invention, the first adhesive layer 230 may be a medically-acceptable, pressure-sensitive adhesive. For example, the first adhesive layer 230 may comprise an acrylic-based adhesive, such as a high-bond strength acrylic adhesive. In some embodiments, not according to the invention the first adhesive layer 230 may be characterized as exhibiting a high bond strength and/or a high tackiness relative to the second adhesive layer 240. For example, the first adhesive layer 230 may have a peel adhesion or resistance to being peeled from about 6 Newtons/25 millimeters (N/mm) to about 10N/25mm from a stainless steel substrate at 23° C at 50% relative humidity as defined by the American Society for Testing and Materials ("ASTM") in standard ASTM D3330.

The first adhesive layer 230 may be continuous or uninterupted. Alternatively, the first adhesive layer 230 may be formed in a pattern and/or may include a plurality of apertures, for example, which may be aligned with the plurality of apertures 225 of the non-adherent layer 220. In such embodiments, the plurality of apertures within the first adhesive layer 230 may be formed after application of the first adhesive layer 230 or may be formed by coating the first adhesive layer 230 in a pattern. Additionally or alternatively, in some embodiments, the plurality of apertures may be sized to control the resultant tackiness of the first adhesive layer 230.

In some embodiments, not according to the invention the second adhesive layer 240 may be coupled to the non-adherent layer 220, for example, to the first surface 222 of the non-adherent layer 220. In some embodiments, not according to the invention the second adhesive layer 240 may have a thickness in the range from about 100 microns (µm) to about 1000 microns (µm). Additionally or alternatively, in some embodiments, not according to the invention the second adhesive layer 240 may have a coating weight of about 30 gsm to about 70 gsm.

In some embodiments, the second adhesive layer 240 may be a relatively soft material capable of adhering two manifold modules. The second adhesive layer 240 comprises a silicone adhesive, for example, including a silicone elastomer. Suitably, the silicone adhesive may be a soft skin adhesive. In some embodiments, silicone adhesives may be made by an addition reaction, for example, a hydrosilylation reaction, between (a) a vinyl functional polydimethyl siloxane, such as bis-dimethyl vinyl polydimethylsiloxane (PDMS), and (b) a hydrogen functional siloxane, such as dimethyl, methylhydrogen siloxane copolymers, or hydrogen dimethylsiloxy terminated PDMS. The reaction may be catalyzed by a hydrosilylation catalyst, for example, a noble metal catalyst such as a platinum catalyst.

In some embodiments, the second adhesive layer 240 may have a stiffness between about 5 Shore OO and about 80 Shore OO. In some embodiments, the second adhesive layer 240 may be characterized as exhibiting a low to medium bond strength and/or a low to medium tackiness, relative to the first adhesive layer 230. For example, the second adhesive layer 240 may have a peel adhesion or resistance to being peeled from about 0.5N/25mm to about 1.5N/25mm from a stainless steel substrate at 23° C at 50% relative humidity as defined in standard ASTM D3330. The second adhesive layer 240 may achieve its bond strength after a contact time of less than 60 seconds. In some embodiments, the second adhesive layer 240 may exhibit a tackiness from about 30% to about 50% of the tackiness exhibited by the first adhesive layer 230.

In some embodiments, the second adhesive layer 240 may be continuous or uninterupted. Alternatively, in some embodiments the second adhesive layer 240 may be formed in a pattern and/or may include a plurality of apertures, for example, which may be aligned with the plurality of apertures 225 of the non-adherent layer 220. The apertures may be numerous shapes, for example, circles, squares, stars, ovals, polygons, slits, complex curves, rectilinear shapes, triangles, or other shapes. The apertures may have a uniform pattern or may be randomly distributed. The average effective diameter of each of the apertures may be in the range from about 6 mm to about 50 mm.

### Manifold Modules - Arrangement

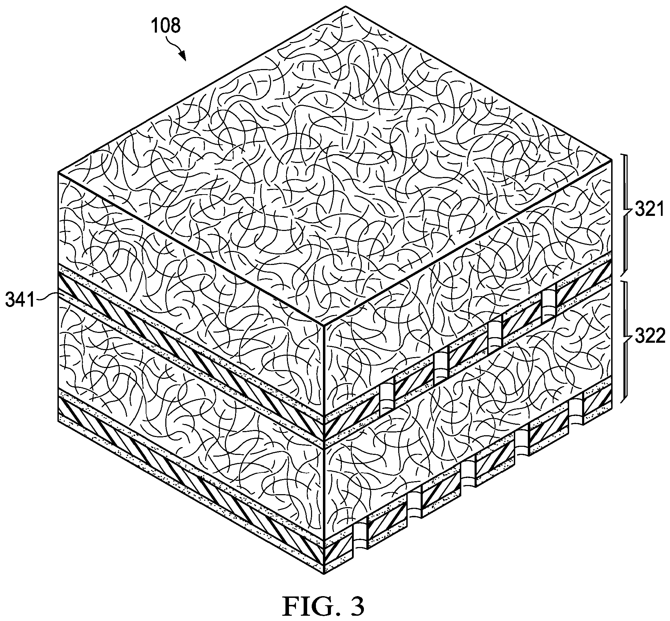

At least two of manifold modules are releasably secured to each other. For example, the second adhesive layer 240 allows a first manifold module to be releasably secured with respect to a second manifold module. As illustrated in Figure 3, a first manifold module 321 including a second adhesive layer 341 is coupled to a second manifold module 322 via the second adhesive layer 341 of the first manifold module 321. Additionally, the second adhesive layer 341 of the first manifold module 321 allows the second manifold module 322 to be released from the first manifold module 321 such that the second adhesive layer 341 of the first manifold module 321 may be later coupled to another manifold module. For example, after being disengaged, the second adhesive layer 341 of the first manifold module 321 may be coupled to the second manifold module 322 or a third manifold module.

In various embodiments, a tissue interface having one or more desired parameters may be achieved by arranging and coupling a suitable combination of manifold modules. For example, by coupling various, suitable manifold modules, a tissue interface having a desired thickness, a desired cross-sectional profile or shape, a desired surface area, or combinations thereof may be achieved. In some embodiments, any two manifold modules of a tissue interface may be similarly configured in at least one respect. For example, two or more manifold modules of a tissue interface may have the same or a similar physical dimension, the same or similar components, or the same or a similar arrangement of components. Additionally or alternatively, in some embodiments, two or more manifold modules of a tissue interface may have differing physical dimensions, the differing components, or a differing arrangement of components. For example, a first manifold module may include a manifold layer having a first thickness and a second manifold module may include a manifold layer having a second thickness.

Also, in some embodiments not according to the invention, a kit for forming a dressing interface may include two, three, four, five, six, seven, eight, nine, ten, or more manifold modules. In some embodiments, the manifold modules of the kit may be sterile and may be packaged in a microorganism-impermeable container. In some embodiments, one or more manifold modules may be individually packaged. Additionally or alternatively, in some embodiments one or more removable protective sheets may be applied to the second adhesive layer of a manifold module while packaged. The protective sheets may be removed by a physician or other caregiver when ready for use.

### Methods

In operation, the tissue interface 108 may be prepared for use in a therapy by selecting two or more manifold modules that, when coupled, will impart the desired properties, for example, the desired physical dimensions. In some embodiments, not according to the invention the tissue interface 108 may be prepared from a kit including a plurality of manifold modules 125. In some embodiments not according to the invention, preparing the tissue interface 108 may include repositioning a selected manifold module with respect to another selected manifold module. For example, a plurality of manifold modules may be provided together. In some embodiments, not according to the invention the manifold modules 125 may be provided in a kit or packaging where several manifold modules may be releasably coupled together. In such embodiments not according to the invention, preparing the tissue interface 108 may include uncoupling a manifold module from one or more other manifold modules, rearranging the selected manifold modules, such as by coupling two or more selected manifold modules that were not previously coupled to yield the tissue interface 108. The selected manifold modules may be coupled to yield the tissue interface 108.

When prepared, the tissue interface 108 may be placed within, over, on, or otherwise proximate to a tissue site. The cover 106 may be placed over the tissue interface 108 and sealed to an attachment surface near the tissue site. For example, the cover 106 may be sealed to undamaged epidermis peripheral to a tissue site. Thus, the dressing 102 can provide a sealed therapeutic environment proximate to a tissue site, substantially isolated from the external environment, and the negative-pressure source 104 can reduce the pressure in the sealed therapeutic environment. Negative pressure applied across the tissue site through the tissue interface 108 in the sealed therapeutic environment can induce macrostrain and microstrain in the tissue site, as well as remove exudates and other fluids from the tissue site, which can be collected in container 112.

### Advantages

In various embodiments, a therapy system like therapy system 100 or components thereof, such as the tissue interface 108, may be advantageously employed in the provision of therapy, such as negative pressure therapy, to a patient. For example, a tissue interface having one or more desired and/or customized parameters, such as a desired thickness, a desired cross-sectional profile or shape, a desired surface area, or combinations thereof, may be achieved. As such, a tissue interface formed from a combination of manifold modules may yield a desired and/or customized parameters without the need to cut or manipulate the structure of a dressing.

Additionally, a second adhesive layer, such as a silicone adhesive, applied to a non-adherent layer, such as a nylon layer, may be effective both to couple two manifold modules together and to adhere the tissue interface to a tissue site, such as a wound bed. The silicone adhesive may allow for ease of application to the tissue site and ease of removal after use. For example, the silicone adhesive applied to a nylon layer may create a barrier to tissue in-growth and integration.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the container 112, or both may be eliminated or separated from other components for manufacture or sale. In other example configurations, the controller 110 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

## Claims

1. A system comprising:
a first manifold module comprising:
a first manifold layer, and
a first silicone adhesive layer; and
a second manifold module configured to be releasably secured to the first manifold module by the first silicone adhesive layer and comprising:
a second manifold layer, and
a second silicone adhesive layer,
wherein the first and second manifold modules each comprise a plurality of fluid pathways to distribute reduced pressure from a reduced-pressure source to a tissue site.

2. The system claim 1, wherein the first manifold layer comprises a hydrophobic foam.

3. The system of one of claims 1-2, wherein the first manifold layer comprises a polyurethane foam.

4. The system of one of claims 1-3, wherein the second manifold layer comprises a hydrophobic foam or a polyurethane foam.

5. The system of one of claims 1-4, wherein the first manifold layer and the second manifold layer are substantially the same thickness.

6. The system of one of claims 1-5, wherein the first manifold layer and the second manifold layer have different thicknesses.

7. The system of one of claims 1-6, wherein the first manifold module further comprises:
a first perforated substrate; and
a first acrylic adhesive layer adhering the first manifold layer to a first surface of the first perforated substrate;
wherein the first silicone adhesive layer is disposed on a second surface of the first perforated substrate.

8. The system claim 7, wherein the second manifold module further comprises:
a second perforated substrate; and
a second acrylic adhesive layer adhering the second manifold layer to a first surface of the second perforated substrate;
wherein the second silicone adhesive layer is disposed on a second surface of the second perforated substrate.

9. The system of one of claims 7-8, wherein the first perforated substrate comprises nylon, and wherein the second perforated substrate comprises nylon.

10. The system of one of claims 1-9, wherein at least one of the first silicone adhesive layer and the second silicone adhesive layer is non-continuous.

11. The system of one of claims 1-10, wherein the dressing further comprises a third manifold module releasably secured to the second manifold module by the second silicone adhesive layer and comprising:
a third manifold layer; and
a third silicone adhesive layer.

12. The system of claim 11, wherein the third manifold module further comprises:
a third perforated substrate; and
a third acrylic adhesive layer adhering the third manifold layer to a first surface of the third perforated substrate;
wherein the third silicone adhesive layer is disposed on a second surface of the third perforated substrate.

13. The system of one of claims 1-12, further comprising the reduced-pressure source adapted to be fluidly coupled to the dressing.

14. The system of one of claims 7-13, wherein the first silicone adhesive layer has a bond strength and the first acrylic adhesive layer has a bond strength, wherein the bond strength of the first acrylic adhesive layer is greater than the bond strength the first silicone adhesive layer.

15. The system of one of claims 8-14, wherein the second silicone adhesive layer has a bond strength and the second acrylic adhesive layer has a bond strength, wherein the bond strength of the second acrylic adhesive layer is greater than the bond strength the second silicone adhesive layer.

## Patentansprüche

1. Ein System, aufweisend:
ein erstes Verteilermodul, aufweisend:
eine erste Verteilerschicht und
eine erste Silikonklebstoffschicht; und
ein zweites Verteilermodul, das konfiguriert ist, um durch die erste Silikonklebstoffschicht an dem ersten Verteilermodul lösbar befestigt zu sein, und aufweisend:
eine zweite Verteilerschicht und
eine zweite Silikonklebstoffschicht,
wobei das erste und das zweite Verteilermodul jeweils eine Vielzahl von Fluidwegen aufweisen, um Unterdruck von einer Unterdruckquelle zu einer Gewebestelle zu verteilen.

2. Das System nach Anspruch 1, wobei die erste Verteilerschicht einen hydrophoben Schaum aufweist.

3. Das System nach einem der Ansprüche 1 bis 2, wobei die erste Verteilerschicht einen Polyurethanschaum aufweist.

4. Das System nach einem der Ansprüche 1 bis 3, wobei die zweite Verteilerschicht einen hydrophoben Schaum oder einen Polyurethanschaum aufweist.

5. Das System nach einem der Ansprüche 1 bis 4, wobei die erste Verteilerschicht und die zweite Verteilerschicht im Wesentlichen die gleiche Dicke aufweisen.

6. Das System nach einem der Ansprüche 1 bis 5, wobei die erste Verteilerschicht und die zweite Verteilerschicht unterschiedliche Dicken aufweisen.

7. Das System nach einem der Ansprüche 1 bis 6, wobei das erste Verteilermodul ferner aufweist:
ein erstes perforiertes Substrat; und
eine erste Acrylklebstoffschicht, die die erste Verteilerschicht an eine erste Oberfläche des ersten perforierten Substrats klebt;
wobei die erste Silikonklebstoffschicht auf einer zweiten Oberfläche des ersten perforierten Substrats angeordnet ist.

8. Das System nach Anspruch 7, wobei das zweite Verteilermodul ferner aufweist:
ein zweites perforiertes Substrat; und
eine zweite Acrylklebstoffschicht, die die zweite Verteilerschicht an eine erste Oberfläche des zweiten perforierten Substrats klebt;
wobei die zweite Silikonklebstoffschicht auf einer zweiten Oberfläche des zweiten perforierten Substrats angeordnet ist.

9. Das System nach einem der Ansprüche 7 bis 8, wobei das erste perforierte Substrat Nylon aufweist und wobei das zweite perforierte Substrat Nylon aufweist.

10. Das System nach einem der Ansprüche 1 bis 9, wobei mindestens eine der ersten Silikonklebstoffschicht und der zweiten Silikonklebstoffschicht nicht fortlaufend ist.

11. Das System nach einem der Ansprüche 1 bis 10, wobei der Verband ferner ein drittes Verteilermodul aufweist, das durch die zweite Silikonklebstoffschicht an dem zweiten Verteilermodul lösbar befestigt ist und aufweisend:
eine dritte Verteilerschicht; und
eine dritte Silikonklebstoffschicht.

12. Das System nach Anspruch 11, wobei das dritte Verteilermodul ferner aufweist:
ein drittes perforiertes Substrat; und
eine dritte Acrylklebstoffschicht, die die dritte Verteilerschicht an eine erste Oberfläche des dritten perforierten Substrats klebt;
wobei die dritte Silikonklebstoffschicht auf einer zweiten Oberfläche des dritten perforierten Substrats angeordnet ist.

13. Das System nach einem der Ansprüche 1 bis 12, ferner aufweisend die Unterdruckquelle, die angepasst ist, um mit dem Verband fluidisch gekoppelt zu sein.

14. Das System nach einem der Ansprüche 7 bis 13, wobei die erste Silikonklebstoffschicht eine Haftfestigkeit aufweist und die erste Acrylklebstoffschicht eine Haftfestigkeit aufweist, wobei die Haftfestigkeit der ersten Acrylklebstoffschicht größer als die Haftfestigkeit der ersten Silikonklebstoffschicht ist.

15. Das System nach einem der Ansprüche 8 bis 14, wobei die zweite Silikonklebstoffschicht eine Haftfestigkeit aufweist und die zweite Acrylklebstoffschicht eine Haftfestigkeit aufweist, wobei die Haftfestigkeit der zweiten Acrylklebstoffschicht größer als die Haftfestigkeit der zweiten Silikonklebstoffschicht ist.

## Revendications

1. Système comprenant :
un premier module de collecteur comprenant :
une première couche de collecteur, et
une première couche adhésive en silicone ; et
un deuxième module de collecteur conçu pour être assujetti de manière amovible au premier module de collecteur par la première couche adhésive en silicone et comprenant :
une deuxième couche de collecteur, et
une deuxième couche adhésive en silicone,
dans lequel les premier et deuxième modules de collecteur comprennent chacun une pluralité de trajets de fluide pour distribuer une pression réduite à partir d'une source de pression réduite à un site tissulaire.

2. Système selon la revendication 1, dans lequel la première couche de collecteur comprend une mousse hydrophobe.

3. Système selon l'une des revendications 1 à 2, dans lequel la première couche de collecteur comprend une mousse de polyuréthane.

4. Système selon l'une des revendications 1 à 3, dans lequel la deuxième couche de collecteur comprend une mousse hydrophobe ou une mousse de polyuréthane.

5. Système selon l'une des revendications 1 à 4, dans lequel la première couche de collecteur et la deuxième couche de collecteur ont sensiblement la même épaisseur.

6. Système selon l'une des revendications 1 à 5, dans lequel la première couche de collecteur et la deuxième couche de collecteur ont des épaisseurs différentes.

7. Système selon l'une des revendications 1 à 6, dans lequel le premier module de collecteur comprend en outre :
un premier substrat perforé ; et
une première couche adhésive acrylique faisant adhérer la première couche de collecteur à une première surface du premier substrat perforé ;
dans lequel la première couche adhésive en silicone est disposée sur une seconde surface du premier substrat perforé.

8. Système selon la revendication 7, dans lequel le deuxième module de collecteur comprend en outre :
un deuxième substrat perforé ; et
une deuxième couche adhésive acrylique faisant adhérer la deuxième couche de collecteur à une première surface du deuxième substrat perforé ;
dans lequel la deuxième couche adhésive en silicone est disposée sur une seconde surface du deuxième substrat perforé.

9. Système selon l'une des revendications 7 à 8, dans lequel le premier substrat perforé comprend du nylon, et dans lequel le deuxième substrat perforé comprend du nylon.

10. Système selon l'une des revendications 1 à 9, dans lequel au moins l'une de la première couche adhésive en silicone et de la deuxième couche adhésive en silicone est non continue.

11. Système selon l'une des revendications 1 à 10, dans lequel le pansement comprend en outre un troisième module de collecteur assujetti de manière amovible au deuxième module de collecteur par la deuxième couche adhésive en silicone et comprenant :
une troisième couche de collecteur ; et
une troisième couche adhésive en silicone.

12. Système selon la revendication 11, dans lequel le troisième module de collecteur comprend en outre :
un troisième substrat perforé ; et
une troisième couche adhésive acrylique faisant adhérer la troisième couche de collecteur à une première surface du troisième substrat perforé ;
dans lequel la troisième couche adhésive en silicone est disposée sur une seconde surface du troisième substrat perforé.

13. Système selon l'une des revendications 1 à 12, comprenant en outre la source à pression réduite conçue pour être accouplée de manière fluidique au pansement.

14. Système selon l'une des revendications 7 à 13, dans lequel la première couche adhésive en silicone a une force de liaison et la première couche adhésive acrylique a une force de liaison, dans lequel la force de liaison de la première couche adhésive acrylique est supérieure à la force de liaison de la première couche adhésive en silicone.

15. Système selon l'une des revendications 8 à 14, dans lequel la deuxième couche adhésive en silicone a une force de liaison et la deuxième couche adhésive acrylique a une force de liaison, dans lequel la force de liaison de la deuxième couche adhésive acrylique est supérieure à la force de liaison de la deuxième couche adhésive en silicone.
